(19) Europäisches Patentamt
European Patent Office
Office européen des brevets

(11) Publication number: **0 258 182 A1**

(12) **EUROPEAN PATENT APPLICATION**

(21) Application number: **87810444.7**

(22) Date of filing: **03.08.87**

(51) Int. Cl.[4]: **C 07 D 275/02**
C 07 D 277/30, C 07 D 277/34, C 07 D 285/06, C 07 D 231/16, C 07 D 213/80, C 07 D 213/82, C 07 D 213/83, C 07 D 263/32, C 07 D 263/38, C 07 D 263/48

(30) Priority: **08.08.86 US 894990 02.02.87 US 9963**

(43) Date of publication of application: **02.03.88 Bulletin 88/09**

(84) Designated Contracting States: **AT BE CH DE ES FR GB GR IT LI NL**

(71) Applicant: **SANDOZ AG**
**Lichtstrasse 35**
**CH-4002 Basel (CH)**

(84) Designated Contracting States: **BE CH ES FR GB GR IT LI NL**

(71) Applicant: **SANDOZ-PATENT-GMBH**
**Humboldtstrasse 3**
**D-7850 Lörrach (DE)**

(84) Designated Contracting States: **DE**

(71) Applicant: **SANDOZ-ERFINDUNGEN Verwaltungsgesellschaft m.b.H.**
**Brunner Strasse 59**
**A-1235 Wien (AT)**

(84) Designated Contracting States: **AT**

(72) Inventor: **Anderson, Richard James**
**3376 Kenneth Drive**
**Palo Alto California 94303 (US)**

**Leippe, Michael Mario**
**230 Madrone Avenue**
**Boulder Creek California 95006 (US)**

**Bamberg, Joe T.**
**258 Hillsdale Way**
**Redwood City California 94062 (US)**

(54) **Semicarbazones and thiosemicarbazones.**

(57) The invention relates to novel compounds of formula (A)

$$\underset{\textstyle R^3}{R - \overset{}{C}} = N - \underset{\textstyle R^4}{N} - R^2 \qquad (A)$$

wherein $R^2$ is the group $-C(=NR^{10})-SR^9$ or $-C(=X)-NHR^{10}$ and R, $R^3$, $R^4$, $R^9$ and $R^{10}$ are as stated in the specification, the synthesis thereof, the use of said compounds for the control of weeds and compositions for weed control comprising such compounds.

EP 0 258 182 A1

**Description**

## SEMICARBAZONES AND THIOSEMICARBAZONES

This invention relates to novel substituted semicarbazones, thiosemicarbazones, and isothiosemicarbazones, the synthesis thereof, the use of said compounds for the control of weeds, and compositions for weed control comprising such compounds.

The compounds of the present invention are represented by the following formula (A):

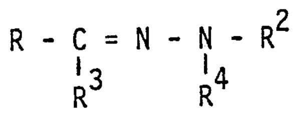 (A)

wherein R is an heteroaromatic group selected from the groups $G_1$ to $G_9$,

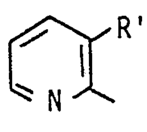
($G_1$)

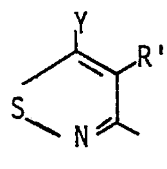
($G_2$)

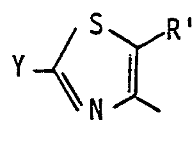
($G_3$)

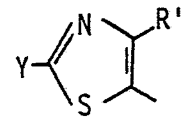
($G_4$)

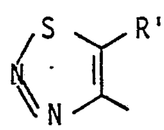
($G_5$)

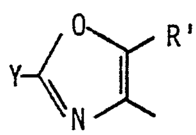
($G_6$)

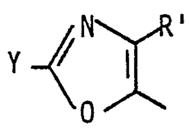
($G_7$)

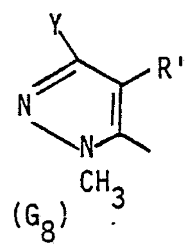
($G_8$)

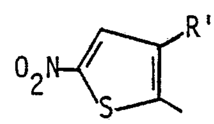
($G_9$)

in which R′ is the carboxy group in free form, salt form or ester form or is the group CO-$SR^6$ or CO-$NR^7R^8$, and

Y is H, $C_{1-8}$alkyl, $C_{1-8}$haloalkyl, $C_{1-8}$alkoxy, $C_{1-8}$haloalkoxy, $C_{2-8}$alkenyloxy, $C_{2-8}$haloalkenyloxy, $C_{2-8}$alkynyloxy, phenyl, phenoxy, $C_{2-5}$alkenyl, $C_{1-8}$alkylthio, OH, halogen, nitro or cyano,

$R^2$ is the group $-C(=NR^{10})-SR^9$ or $-C(=X)-NHR^{10}$,

$R^3$ is H or $C_{1-8}$alkyl,

$R^4$ is H, $C_{1-8}$alkyl or 2-hydroxyethyl,

$R^7$ and $R^8$, independently, are H or $C_{1-8}$alkyl or

$R^7$ together with $R^8$ is $-(CH_2)_2-O-(CH_2)_2-$,

each of $R^6$ and $R^9$ is, indepently, $C_{1-8}$alkyl, $C_{2-8}$alkenyl, phenyl or benzyl,

X is oxygen or sulfur,

$R^{10}$ is one of the groups

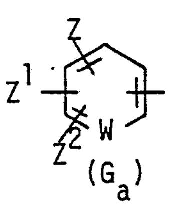
($G_a$)

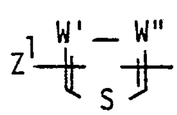
($G_b$)

in which W, W′ and W″, independently, are N or CH,

and

Z, $Z^1$ and $Z^2$, independently, are one of the significances specified for - but independent of - Y with the provisos that either

a) where R is a nicotinyl acid group in free acid form, salt form or ester form then $R^2$ is the group $-C(=NR^{10})-SR^9$ or

b) where R is a nicotinyl acid group in free acid form, salt form or ester form, and $R^2$ is the group $-C(=X)-NHR^{10}$, then $R^{10}$ is the group ($G_b$), or

c) where R is a nicotinyl acid group in free acid form, salt form or ester form, $R^2$ is the group $-C(=X)-NHR^{10}$ and $R^{10}$ is the group ($G_a$), then either

i) one of Z, $Z^1$ and $Z^2$ is selected from $C_{2-5}$alkenyl, $C_{1-8}$alkylthio and $C_{1-8}$haloalkoxy,

or

ii) $Z^1$ is $C_{1-8}$alkyl, $Z^2$ is halogen or $C_{1-8}$alkyl and Z is H, or

iii) $Z^1$ and $Z^2$ are both bromo, and Z is H, or

iv) $R^4$ is 2-hydroxyethyl.

The terms $C_{2-8}$alkenyl-O and $C_{2-8}$alkenyl-O when used herein refer to $C_{2-8}$hydrocarbyloxy groups having I or 2, preferably I, ethylenic bonds or I or 2, preferably I, acetylenic bonds resp.

The terms $C_{1-8}$haloalkyl, $C_{1-8}$alkoxy and $C_{2-8}$haloalkenyloxy refer to $C_{1-8}$alkyl, $C_{1-8}$alkoxy and $C_{2-8}$alkenyloxy resp. substituted by one to six halogen.

A preferred $C_{1-8}$haloalkyl group is $CF_3$.

Preferred $C_{1-8}$haloalkoxy groups are $C_{1-4}$haloalkoxy groups and particularly $OCF_3$, $OCHF_2$ and $OCF_2CHF_2$.

Where R′ is the carboxyl group in salt form, its cation is, for example, the cation of an alkali metal (e.g. the Li or Na cation) or of an earth alkali metal (e.g. the Ca cation); the ammonium cation; a substituted ammonium cation (such as $C_{1-20}$alkylammonium cation, a di-$C_{1-5}$-alkylammonium cation, a tri-$C_{1-5}$alkylammonium cation, a tetra-$C_{1-5}$alkylammonium cation, a ($C_{1-5}$alkoxy-alkyl)ammonium cation, a (hydroxy-$C_{1-5}$-alkoxy-$C_{1-5}$alkyl)ammonium cation, a (hydroxy-$C_{1-5}$alkyl)ammonium carbon); a phosphonium cation; a tri($C_{1-8}$alkyl)sulfonium cation or a tri($C_{1-8}$-alkyl)-sulfoxonium cation; a hydroxy-$C_{1-5}$alkylammonium cation or a di- or tri-(hydroxy-$C_{1-5}$alkyl)ammonium cation.

Examples of preferred ammonium cations are the isopropyl-, octyl-, 2-(2-hydroxyethoxy)ethyl-, 2-hydroxyethyl-, dimethyl-, di-2-hydroxyethl-and the tri-2-hydroxyethyl-ammonium cation.

Where R′ is a carboxy ester group (hereinafter $COOR^5$), then $R^5$ is for example $C_{1-8}$alkyl, $C_{1-8}$haloalkyl, $C_{2-10}$alkoxyalkyl, or $CH(R^{11})-O-C(=X')-R^{12}$, wherein X′ is O or S, and $R^{11}$ and $R^{12}$ are, independently, H or $C_{1-8}$alkyl.

$R^5$ is preferably $C_{1-4}$alkyl, phenyl, benzyl, allyl, $C_{1-4}$haloalkyl or $CH(R^{11})O-C(O)-R^{12}$ in which $R^{11}$ is H or $CH_3$ and $R^{12}$ is $C_{1-8}$alkyl.

Where any of $R^3$, $R^4$, $R^5$, $R^6$, $R^7$, $R^8$, $R^9$, $R^{10}$, $R^{11}$, $R^{12}$, Y, Z, $Z^1$, $Z^2$ or the cation of the carboxy group R′ is or comprises $C_{1-8}$alkyl, such alkyl group or moiety comprises preferably $C_{1-5}$, more preferably $C_{1-4}$e.g. I or 2 carbon atoms.

The term $C_{1-8}$alkylthio refers preferably to $C_{1-4}$alkylthio, particularly $CH_3S$.

The term $C_{2-5}$alkenyl refers to hydrocarbyl groups having I ethylenic bond. Examples of prefered $C_{2-5}$ alkenyl groups are $C(CH_3)=CH_2$ and allyl.

Y is preferably H, halogen, $C_{1-4}$alkyl or $C_{1-4}$alkoxy. R is preferably group $G_2$

W is preferably CH.

Z is preferably H.

$Z^1$ is preferably H, $C_{1-8}$alkyl, $C_{2-5}$alkenyl, $C_{1-8}$alkylthio, $C_{1-8}$- alkoxy, $C_{1-8}$haloalkoxy, halogen (F, Cl, Br, I) or $CF_3$.

$Z^2$ is preferably H, $C_{1-4}$alkyl, halogen or $C_{1-4}$alkoxy.

Preferred groups $G_b$ are 2-thiazole and 5-($C_{1-4}$alkyl)-2-(l,3,4-thiadiazole).

R′ is preferably the carboxy group in free form, salt form or ester form.

$R^2$ is preferably $CO-NHR^{10}$.

$R^3$ is preferably $CH_3$ or $C_2H_5$.

$R^4$ is preferably H, $CH_3$ or $C_2H_5$.

Accordingly a particularly preferred gorup of compounds of formula (A) is of formula (AA)

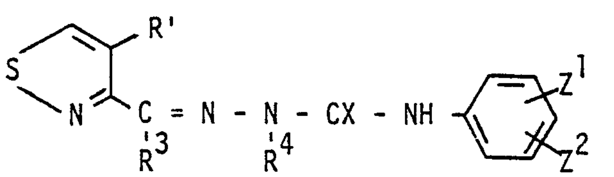

(AA)

in which

R′ is carboxy in free form, salt form or ester form,

$R^3$ is $CH_3$ or $C_2H_5$,

$R^4$ is H, $CH_3$ or $C_2H_5$,

$Z^1$ is H, $C_{1-2}$alkyl, methoxy, halogen (F, Cl, Br, I), $CF_3$ or $OCF_3$,

$Z^2$ is H, halogen (e.g. F, Cl), methyl or methoxy.

Where in formula (AA) R′ is COOH in salt form, preferred significances of its cation are, for example, the sodium, the ammonium, a $C_{1-20}$-alkylammonium, a di-$C_{1-5}$alkylammonium, a hydroxy-$C_{1-5}$alkylammonium, a hydroxy-$C_{1-5}$alkylammonium, a di-(hydroxy-$C_{1-5}$alkyl)ammonium or hydroxy-$C_{1-5}$alkoxy-$C_{1-5}$alkylammonium cation.

Where in the formula (AA) R′ is COOH in ester form ($COOR^5$), a preferred significance of $R^5$ is, for example, $CH(R^{11})$-O-$C(O)R^{12}$, particularly such in which $R^{11}$ is H, methyl, ethyl or sec. butyl and $R^{12}$ is H, methyl, ethyl, n-propyl, sec. butyl or tert. butyl).

Other preferred $Z^1$ significances in formula (AA) are $OCHF_2$ and $OCF_2$-$CHF_2$.

Other prefered $R^5$ significances in formula (AA) are $CH_2CF_3$, $C_{1-4}$-alkyl and phenyl. X is preferably O.

The compounds of formula (A) are obtained by

a) reacting a compound of formula I

$R - C(=O)-R^3$ I

wherein R and $R^3$ are as defined above

with a compound of formula II

$H_2N - NR^2R^4$ II

wherein $R^2$ and $R^4$ are as defined above,

followed, where desired, by esterifying compounds of formula (A) wherein R′ is the carboxyl group to compounds (A) wherein R′ is a carboxyl-ester group, or

b) S-alkylating a compound of formula $A_1$

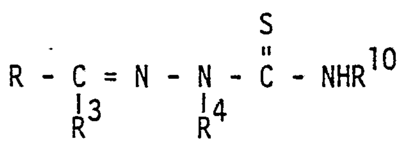

$(A_1)$

wherein R, $R^3$, $R^4$ and $R^{10}$ are as defined above,

with a halogenide of formula III

$R^9Hal$ III

wherein $R^9$ is as defined above and

Hal is halogen,

or a reactive functional derivative of said compound of formula III

to give a compound of formula ($A_2$)

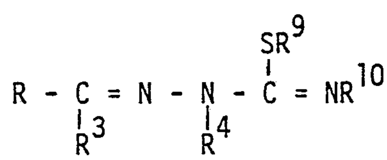

$(A_2)$

wherein R, $R^3$, $R^4$, $R^9$ and $R^{10}$ are as defined above.

The reaction of compounds of formula (I) with compounds of formula (II) may be effected under the conditions known for the preparation of (thio)semicarbazones starting from the corresponding (thio)carbazides.

The reaction is conveniently carried out in a solvent which is inert under the reaction conditions, e.g. in an alcohol such as methanol or ethanol. An acid catalyst such as pyridyl tosylate may be added to promote the reaction. A suitable reaction temperature is room temperature or moderately enhanced temperature (e.g. between IO and 40°C).

Compounds of formula (A) wherein R′ is $COOR^5$ may also be, and are in general preferably obtained by esterification of the corresponding compounds of formula (A) wherein R′ is the carboxyl group, in free form or in salt form, e.g. alkali metal salt form, using the desired esterification agent. Suitable esterification agents are alkylating agents such as $R^5$-halogenides, $R^5$-mesylates and $R^5$-tosylates or reactive functional derivatives thereof such as $CH_2N_2$.

The S-alkylation of thiosemicarbazones of formula ($A_1$) may be effected under the conditions known for the preparation of isothiosemicarbazones from thiosemicarbazones. In general, the reaction is carried out in a solvent which is inert under the reaction conditions, e.g. dimethylformamide. The compound of formula III is conveniently used as an iodide. It is in general advantageous to work in the presence of an acid binding agent, such as potassium carbonate. The R′ group may partially or completely be esterified or its eventual $R^5$ group interchanged, depending on the particular reaction conditions employed. Hydrolysis of such ester group followed, where desired, by treatment with an esterification (e.g. alkylation) agent for the introduction of the selected groupb $R^5$, will then result in the desired compound of formula ($A_2$).

Compounds of formula (A) wherein $R^1$ is carboxy may be converted to the corresponding salts in conventional manner and vice versa.

The compounds of formula (A) may be recovered from the reaction mixture in which they are formed by working up by established procedures.

The compounds of formula (A) may exist in either the syn or the anti form, although the anti form usually

predominates.

The isothiosemicarbazones of the invention (see formula ($A_2$)) wherein $R^4$ is hydrogen may also exist in the corresponding isomeric form

$RR^3$-C = N-N = C($SR^9$)-$NHR^{10}$.

The starting materials and reagents employed in the process described herein are either known or, insofar as they are not known, may be produced in a manner analogous to the process described herein or to known processes.

The compounds of formula (A) have herbicidal activity as observed after their pre-emergent or post-emergent application to weeds or a weed locus.

The term "herbicide" (or "herbicidal") refers to an active ingredient (or an effect) which modifies the growth of plants because of plant growth regulating or phytotoxic properties so as to retard the growth of the plant or damage the plant sufficiently to kill it.

Application of a compound of formula (A) is made according to conventional procedure to the weeds or their locus using a herbicidally effective amount of the compound, usually from 100 g to 10 kg/ha.

The optimum usage of a compound of formula (A) is readily determined by one of oridinary skill in the art using routine testing such as greenhouse testing and small plot testing. It will depend on the compound employed, the desired effect (a phytotoxic effect requiring a higher rate than a plant growth regulating effect), the conditions of treatment and the like. In general satisfacotry phytotoxic effects are obtained when the compound of formula (A) is applied at a rate in the range of from 0.2 to 5.0 kg, more preferably of from 0.25 to 2.5 kg per hectare.

While some of the compounds of formula (A), have activity on grass weeds, they demonstrate, in general, a higher level of herbicidal activity on broadleaf plants when applied post-emergence. Broadleaf plant (weed) species on which the compounds of the present invention show effective herbicidal activity include Brassica juncea, Amaranthus retroflexus, Abutilon theophrasti, Datura stramonium, Xanthium canadense, Cassia obtusifolia and Ipomoea purpurea.

When applied pre-emergence, the compounds of formula (A) demonstrate high levels of herbicidal activity on both broadleaf and grassy weeds.

The compounds of formula A in which R is an optionally substituted isothiazole or optionally substituted pyridine are particularly indicated for selective control of weeds in certain crop plants. These compounds and especially the isothiazoles are particularly suitable for use as selective herbicides in corn. The isothiazoles also show selectivity in soybean locus.

The compounds of formula (A) may be advantageously combined with other herbicides for broadspectrum weed control. Examples of herbicides which can be combined with a compound of the present invention include those selected from the carbamates, thiocarbamates, chloroacetamides, dinitroanilines, benzoic acids, glycerol ethers, pyridazinones, uracils and ureas for controlling a broad spectrum of weeds.

The compounds of formula (A) are conveniently employed as herbicidal compositions in association with agriculturally acceptable diluents. Such compositions also form part of the present invention. They may contain, aside from a compound of formula (A) as active agent, other active agents, such as herbicides. They may be employed in either solid or liquid forms e.g. in the form of a wettable powder or an emulsifiable concentrate, incorporating conventional diluents. Such compositions may be produced in conventional manner, e.g. by mixing the active ingredient with a diluent and optionally other formulating ingredients such as surfactants.

The term diluents as used herein means any liquid or solid agriculturally acceptable material which may be added to the active constituent to bring it in an easier or improved applicable form, respectively to a usable or desirable strength of activity. It can for example be talc, kaolin, diatomaceous earth, xylene, or water.

Particularly formulations to applied in spraying forms such as water dispersible concentrates or wettable powders may contain surfactants such as wetting and dispersing agents, e.g. the condensation product of formaldehyde with naphthalene sulphonate, an alkylarylsulphonate, a lignin sulphonate, a fatty alkyl sulphate, an ethoxylated alkylphenol and an ethoxylated fatty alcohol.

In general, the formulations include from 0.01 to 90% by weight of active agent and from 0 to 20% by weight of agriculturally acceptable surfactant, the active agent consisiting either of at least one compound of formula (A) or mixtures thereof with other active agents. Concentrate forms of compositions generally contain between about 2 and 90%, preferably between about 5 and 70% by weight of active agent. Application forms of formulation may for example contain from 0.01 to 20% by weight of active agent.

The following examples are provided to illustrate the practice of the present invention. Temperature is given in degrees Centigrade. "RT" means room temperature. Parts and percentages are by weight. The symbols *, and + when used in connection with melting points mean "gas", "softens" and "decomposes" resp.

Example 1: 3-Acetyl-4-isothiazolecarboxylic acid 4-(3-fluorophenyl)semicarbazone

To a solution of 3-acetyl-4-isothiazolecarboxylic acid (3.08 g, 18.0 mmol) in methanol is added to a solution of 4-(3-fluorophenyl)semicarbazide (3.04 g, 18.0 mmol) in methanol. The mixture is stirred at RT for 3 hours, after which the solid precipitate is collected by filtration, washed with methanol and with ether and dried to give 3-acetyl-4-iso-thiazole-carboxylic acid 4-(3-fluorophenyl)semicarbazone, m.p. 222° (dec.) (compound 13 , Table C).

Treatment of the thus obtained acid with 1 equivalent of a base gives the corresponding salt.

Example 2

Following the procedure of Example I, the following semicarbazone or semicarbazone compounds under Table A to C are prepared from the corresponding semicarbazide or semicarbazide and acetyl compound.

**TABLE A**

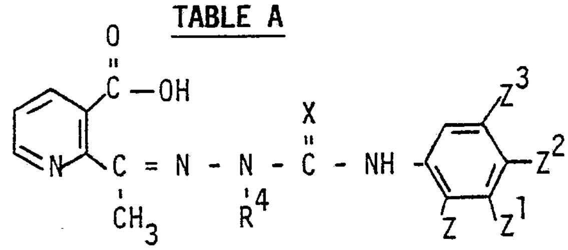

(Formula $A_1$)

| Cpd. No. | $R^4$ | X | Z | $Z^1$ | $Z^2$ | $Z^3$ | m.p. acid | m.p. Na salt |
|---|---|---|---|---|---|---|---|---|
| 1 | H | O | H | $CH_3$ | H | $CH_3$ | 198* 228 | 228* |
| 2 | H | O | H | $C(CH_3)=CH_2$ | H | H | 171-173 | 213+ |
| 3 | H | O | H | Br | H | Br | 202* | |
| 4 | H | O | H | $SCH_3$ | H | H | 174-175* | 208-209 |
| 5 | H | O | H | $OCF_3$ | H | H | 191-192* | 225-227 |
| 6 | H | O | H | $OCHF_2$ | H | H | 193* | 208-212 |
| 7 | H | O | H | $OCF_2CHF_2$ | H | H | | |
| 8 | $CH_2CH_2OH$ | O | H | F | H | H | 146-148* | 80-83* |
| 9 | H | O | H | $CH_3$ | F | H | 222-230 | 220* |

**TABLE B**

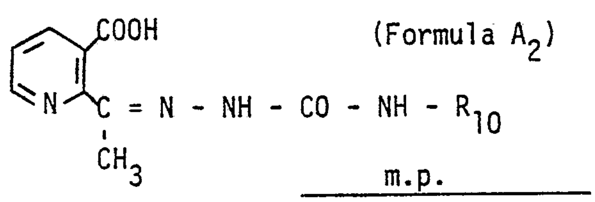

(Formula $A_2$)

| Cpd.no. | $R_{10}$ | m.p. acid | m.p. Na-salt |
|---|---|---|---|
| 10 | 5-methyl-2-(1,3,4-thiazole) | 256-258 | 242-244 |
| 11 | 2-thiazole | 164*,235+ | 219* |
| 12 | 5-ethyl-2-(1,3,4-thiadiazole) | 232-235* | |

## TABLE C

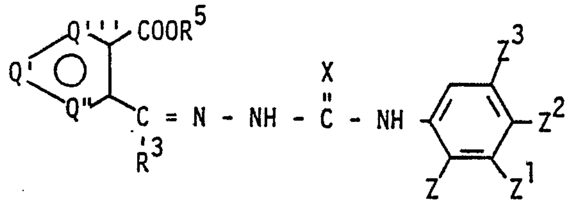

(Formula $A_3$)

| Cpd | Q' | Q'' | Q''' | $R^3$ | $R^5$ | X | Z | $Z^1$ | $Z^2$ | $Z^3$ | acid | Na salt |
|---|---|---|---|---|---|---|---|---|---|---|---|---|
| 13 | S | N | CH | $CH_3$ | H | O | H | F | H | H | 222+ | 202-205* |
| 14 | S | N | CH | $CH_3$ | H | O | H | Cl | H | H | 244-247 | 200-206* |
| 15 | CH | N | S | $CH_3$ | H | O | H | F | H | H | 208-210 | 268-278 |
| 16 | CH | N | S | $CH_3$ | H | O | H | Cl | H | H | | |
| 17 | CH | S | N | $CH_3$ | H | O | H | F | H | H | 187-189 | 236-240+ |
| 18 | N | N | S | $CH_3$ | H | O | H | F | H | H | 205-207+ | 220-230+ |
| 19 | CH | N | O | $CH_3$ | H | O | H | F | H | H | 238-240+ | 265-290+ |
| 20 | $C-NO_2$ | S | CH | $CH_3$ | H | O | H | H | H | H | 200*+ | |
| 21 | $C-NO_2$ | S | CH | $CH_3$ | H | O | H | F | H | H | 205*+ | |
| 22 | S | N | CH | $CH_3$ | H | O | H | H | H | H | 199-204 | 199# |
| 23 | S | N | CH | $CH_3$ | H | O | H | Cl | H | Cl | 235-237+ | 198 |
| 24 | S | N | CH | $CH_3$ | H | O | H | F | H | F | 241-243+ | 238-239+ |
| 25 | S | N | CH | $CH_3$ | H | S | H | Cl | H | H | 188-189+ | 167-172+ |
| 26 | S | N | CH | $CH_3$ | H | S | H | F | H | H | 189-196+ | 164-169+ |
| 27 | S | N | CH | $CH_3$ | H | O | H | $CH_3$ | H | H | 208#,255* | 204-208* |
| 28 | S | N | CH | $CH_3$ | H | O | H | $CH_2CH_3$ | H | H | 201#,260* | 198-201* |
| 29 | S | N | CH | $CH_3$ | H | O | H | Br | H | H | | 197-204+ |
| 30 | S | N | CH | $CH_3$ | H | O | H | I | H | H | | 199-205+ |
| 31 | S | N | CH | $CH_2CH_3$ | H | O | H | F | H | H | 182-183 | 179-183 |
| 32 | S | N | CH | $CH_2CH_3$ | H | O | H | Cl | H | H | 178-183 | 172-178* |
| 33 | S | N | CH | $CH_3$ | $\underset{CH_3}{CH}O\overset{O}{\overset{\|}{C}}\underset{CH_2CH_3}{CH}(CH_2)_3CH_3$ | O | H | F | H | H | | - |
| 34 | S | N | CH | $CH_3$ | $CH_2O\overset{O}{\overset{\|}{C}}C(CH_3)_3$ | O | H | H | H | H | 163-171 | - |

TABLE C (Cont'd)

| Cpd | Q' | Q'' | Q''' | $R^3$ | $R^5$ | X | Z | $Z^1$ | $Z^2$ | $Z^3$ | acid | Na salt |
|---|---|---|---|---|---|---|---|---|---|---|---|---|
| 35 | S | N | CH | $CH_3$ | $CH_2O\overset{O}{\overset{\|}{C}}C(CH_3)_3$ | 0 | H | F | H | H | 159-169 | - |
| 36 | S | N | CH | $CH_3$ | $CH_2O\overset{O}{\overset{\|}{C}}C(CH_3)_3$ | 0 | H | Cl | H | H | 155-165 | - |
| 37 | S | N | CH | $CH_3$ | $CH_2O\overset{O}{\overset{\|}{C}}C(CH_3)_3$ | 0 | H | Cl | H | Cl | 183-194 | - |
| 38 | S | N | CH | $CH_3$ | $C_6H_5$ | 0 | H | F | H | H | | |
| 39 | S | N | CH | $CH_3$ | $CH_2CF_3$ | 0 | H | F | H | H | 200-201 | |
| 40 | S | N | CH | $CH_3$ | H | 0 | H | $OCHF_2$ | H | H | | |
| 41 | S | N | CH | $CH_3$ | H | 0 | H | $OCF_3$ | H | H | | |
| 42 | S | N | CH | $CH_3$ | H | 0 | H | $OCF_2CHF_2$ | H | H | | |
| 43 | CH | O | N | $CH_3$ | H | 0 | H | F | H | H | 252-253+ | |
| 44 | S | N | CH | $CH_3$ | $\underset{CH_3}{CH}O\overset{O}{\overset{\|}{C}}C(CH_3)_3$ | 0 | H | F | H | H | 158-160 | - |
| 45 | S | N | CH | $CH_3$ | $\underset{CH_3}{CH}O\overset{O}{\overset{\|}{C}}\underset{CH_3}{CH}CH_2CH_3$ | 0 | H | F | H | H | 134-135 | - |
| 46 | N | $N-CH_3$ | CH | $CH_3$ | H | 0 | H | F | H | H | 290-293+ | 250-260+ |
| 47 | S | N | CH | $CH_3$ | H | 0 | H | $OCF_3$ | H | H | 218* | 223* |
| 48 | S | N | CH | $CH_3$ | H | 0 | H | $OCHF_2$ | H | H | 204* | 215* |
| 49 | S | N | CH | $CH_3$ | H | 0 | | H | F | H | 243* | 195-201* |
| 50 | S | N | CH | $CH_3$ | H | 0 | Cl | H | H | H | 218* | 208-212 |
| 51 | S | N | CH | $CH_3$ | H | 0 | H | H | Cl | H | 249-253* | 203-209 |
| 52 | S | N | CH | $CH_3$ | H | 0 | H | H | $CH_3$ | H | 237-239 | 216-226+ |
| 53 | S | N | CH | $CH_3$ | H | 0 | H | $OCH_3$ | H | H | 199* | 208-210 |

TABLE C (Cont'd)

| Cpd | Q' | Q'' | Q''' | $R^3$ | $R^5$ | X | Z | $Z^1$ | $Z^2$ | $Z^3$ | acid | Na salt |
|---|---|---|---|---|---|---|---|---|---|---|---|---|
| 54 | S | N | CH | $CH_3$ | H | 0 | $CH_3$ | H | H | H | 276-278 | 194-200 |
| 55 | S | N | CH | $CH_3$ | H | 0 | H | H | $OCH_3$ | H | 195* | 193-203 |
| 56 | S | N | CH | $CH_3$ | H | 0 | $OCH_3$ | H | H | H | 214* | 221* |
| 57 | S | N | CH | $CH_3$ | H | 0 | F | H | H | H | 216* | 246* |
| 58 | S | N | CH | $CH_3$ | H | 0 | H | $CH_3$ | F | H | 222* | 206-220* |
| 59 | S | N | CH | $CH_3$ | H | 0 | H | $CF_3$ | H | H | 276 | 234-235 |
| 60 | S | N | $C-CH_3$ | $CH_3$ | H | 0 | H | F | H | H | 189-192* | 255* |
| 61 | S | N | C-Cl | $CH_3$ | H | 0 | H | F | H | H | 209-210* | 260-268 |
| 62 | S | N | $C-OCH_3$ | $CH_3$ | H | 0 | H | F | H | H | | 193-195* |
| 63 | S | N | CH | $CH_3$ | $CH_2CF_3$ | 0 | H | Cl | H | H | 178-182 | - |
| 64 | S | N | CH | $CH_3$ | $C_6H_5$ | 0 | H | Cl | H | H | 206-208 | - |

Example 3

The sodium salt of Compound 4 is reacted with chloromethyl-2,2-di-methylpropanoate to give t-butylcarbonyloxymethyl-2-acetylnicotinate 4-(3-methylthiophenyl)semicarbazone (m.p. 133-134°) (Compound 65).

TABLE D

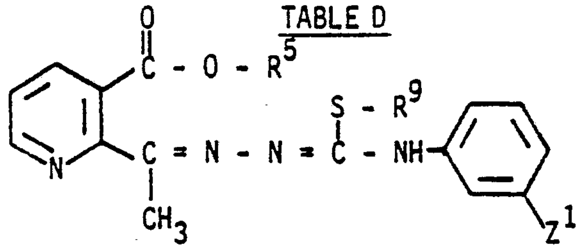

(formula $A_4$)

| Cpd. No. | $R^5$ | $R^9$ | $Z^1$ | m.p.° | m.p.° Na salt | m.p.° $NH_4$ salt |
|---|---|---|---|---|---|---|
| 66 | H | $CH_3$ | H | 140-142* | 110-120#<br>140* | |
| 66(A) | $CH_3$ | $CH_3$ | H | 156-159 | - | - |
| 67 | H | $CH_3$ | Cl | 70#<br>100-110* | 108#,<br>134* | |
| 68 | H | $CH_3$ | F | 80-83 | 155$^{+}$ | 87-90 |
| 69 | H | $CH_2CH_3$ | F | 71-73 | 140-143 | |
| 70 | H | $CH_2CH{=}CH_2$ | F | 126-127 | 157-160 | |
| 71 | H | $CH_2$-$C_6H_5$ | F | 158-159 | 115-117 | |
| 72 | H | $CH_3$ | $CH_3$ | 88$^{+}$ | 64#, 119 | |
| 73 | $CH_2OC(O)C(CH_3)_3$ | $CH_3$ | H | 75-78# | - | - |
| 74 | $CH_2OC(O)C(CH_3)_3$ | $CH_3$ | Cl | (viscous yellow oil) | - | - |
| 75 | $CH_2OC(O)C(CH_3)_3$ | $CH_3$ | F | (oil) | - | - |

Example 4

To a mixture of 2-acetylnicotinic acid 4-phenyl-thiosemicarbazone (8.0 g, 23.4 mmol) and potassium carbonate (6.5 g, 47.0 mmol) in 50 ml of dimethylformamide is added 2.9 ml (6.67 g, 47.0 mmol) of methyl iodide. The mixture is stirred at RT overnight, and is then poured into ether/$CHCl_3$ and water. The organic phase is separated, washed with sodium bicarbonate and with brine and dried and the solvent is removed to give, following crystallization from $CH_3CN$, the corresponding isothiosemicarbazone (compound 66(A), Table D) as a mixture of isomers.

To a suspension of the above ester (5.2 g, 15.2 mmol) in 80 ml of methanol is added a solution of lithium hydroxide monohydrate (0.638 g, 15.2 mmol) in 7 ml of water. The suspension is heated to 60° for 7 days, after which the reaction mixture is cooled and the solid is filtered out. The remaining solution is concentrated by rotoevaporation, and conc. HCl and water are added. The resulting solid is collected by vacuum filtration to give compound 66 under Table D.

Example 5

To a suspension of 2-acetylnicotinic acid 4-(3-chlorophenyl)thiosemicarbazone (15.0 g, 43.1 mmol) in 110 ml of methanol is added sodium methoxide (4.65 g, 86.2 mmol), followed by addition of methyl iodide (6.12 g, 43.1 mmol). The mixture is stirred at RT for 3 hours, after which the solvent is removed by rotoevaporation. The residue is taken up in water and extracted with chloroform. The aqueous fraction is acidified with dilute HCl and extracted with chloroform. The chloroform extract is washed with water and with brine and dried, and the solvent is removed to give Compound 67 under Table D.

Following the above procedures, 2-acetylnicotinic acid 4-(3-fluorophenyl)thiosemicarbazone is reacted with each of methyl iodide, bromoethane, allyl bromide and benzyl bromide to give, respectively, compounds 68-71 under Table D.

Example 6

Following the procedures of Example 5, each of Compounds 25 and 26 is reacted with methyl iodide to give, respectively, 3-acetyl-4-isothiazolecarboxylic acid S-methyl-4-(3-chlorophenyl)isothiosemicarbazone, m.p. 149-153° (Compound 76), and 3-acetyl-4-isothiazolecarboxylic acid S-methyl-4-(3-fluorophenyl)isothiosemicarbazone, m.p. 145-148° (Compound 77).

Each of Compounds 76 and 77 is reacted with sodium methoxide to give:
the sodium salt of compound 76, m.p. 122-130°, and
the sodium salt of compound 77, m.p. 116-136°.

Example 7

The sodium salt of each of compounds 76 and 77 is reacted with chloromethyl 2,2-dimethylpropanoate to give, respectively,

t-butylcarbonyloxymethyl 3-acetyl-4-isothiazolecarboxylate S-methyl-4-(3-chlorophenyl)isothiosemicarbazone, viscous yellow oil (Compound 78), and

t-butylcarbonyloxymethyl 3-acetyl-4-isothiazolecarboxylate S-methyl-4-(3-fluorophenyl)isothiosemicarbazone, m.p. 68-74° (Compound 79).

Example 8

2-Acetylnicotinic acid chloride (1.0 g, 5.5 mmol) and morpholine (0.6 g, 6.7 mmol) are reacted together to give 4-(2-acetylnicotinyl)morpholine, which compound 1.0 g, 4.2 mmol) is then reacted with 4-(3-fluorophenyl)semicarbazide (0.7 g, 4.2 mmol) to give 4-(2-acetyl-nicotinyl)morpholine 4-(3-fluorophenyl)semicarbazone, m.p. 193-195° (Compound 80).

Example 9

Examples of suitable salt forms of compounds of formula (A) are:
of compound
13, the isopropylammonium salt, m.p. 113-122°
67, the isopropylammonium salt, m.p. 98-110°
68, the isopropylammonium salt, m.p. 61-63°
69, the isopropylammonium salt, m.p. 70-73°
70, the isopropylammonium salt, m.p. 57-60°
71, the isopropylammonium salt, m.p. 66-68°
10, the isopropylammonium salt, m.p. 265-269° (dec.)
13, the 2-hydroxyethylammonium salt, m.p. 78-86°
of compound
27, the 2-hydroxyethylammonium salt, m.p. 165° (gas)
28, the 2-hydroxyethylammonium salt, m.p. 75° (softens),95°
29, the 2-hydroxyethylammonium salt, m.p. 82-84°
13, the di-2-hydroxyethylammonium salt, m.p. 174°
14, the di-2-hydroxyethylammonium salt, m.p. 168°
67, the di-2-hydroxyethylammonium salt, m.p. 108° (softens), 112° (dec.)
22, the di-2-hydroxyethylammonium salt, m.p. 162-168°
27, the di-2-hydroxyethylammonium salt m.p. 168° (gas)
28, the di-2-hydroxyethylammonium salt, m.p. 148-151°
29, the di-2-hydroxyethylammonium salt, m.p. 168-170°
31, the di-2-hydroxyethylammonium salt, m.p. 48-70° (sinters), 157-161°
68, the di-2-hydroxyethylammonium salt, m.p. 128-130°
70, the di-2-hydroxyethylammonium salt, m.p. 177-179° (dec.)
77, the di-2-hydroxyethylammonium salt, m.p. 137-140°
13, the tri-2-hydroxyethylammonium salt, m.p. 140° (gas)
13, the 2-(2-hydroxyethoxyl)ethylammonium salt, m.p. 150°
13, the dimethylammonium salt, m.p. 163-165°
14, the dimethylammonium salt, m.p. 230°
67, the dimethylammonium salt, m.p. 138-140° (gas)
68, the dimethylammonium salt, m.p. 53-55°
76, the dimethylammonium salt, m.p. 145-153°
13, the octylammonium salt, m.p. 245-255°

**INTERMEDIATES**

Example IO

Ethyl 3-amino-2-pentenoate (2O g) in 45 ml dimethylformamide is chilled to -78°. Phosphorous oxychloride (25.7g) is added, keeping the temperature between -IO and -3O°. A solid forms and the mixture is then diluted with I2 ml methylene chloride. The resulting mixture is rapidly added to a chilled solution of 5I g sodium sulfide nonahydrate in I5O ml water. Methylene chloride is added to the orange mixture, and the mixutre is stirred I hr at room temperature. I2.5% Aqueous sodium hypochloride (98 ml) is added, and is backwashed with water and with aqueous sodium thiosulfate, dried, filtered and stripped. The residue is distilled to yield ethyl 3-ethyl-4-isothiazolecarboxylate.

Example II

Ethyl 3-ethyl-4-isothiazolecarboxylate is reacted with N-bromosuccinimide (NBS) and benzoyl peroxide to give ethyl 3-(I-bromoethyl)-4-iso-thiazolecarboxylate, which is then reacted with sodium bicarbonate and dimethylsulfoxid and the resulting ethyl 3-acetyl-4-isothiazolecarboxylic acid.

Example I2

Methyl 4-ethyl-5-thiazolecarboxylate is reacted with NBS and 2,2′-azobis(2-methylpropionitrile) to give methyl 4-(I-bromoethyl)-5-thiazolecarboxylate, which is then reacted with sodium bicarbonate, or alternatively with potassium carbonate, and dimethylsulfoxide and the resulting methyl 4-acetyl-5-thiazolecarboxylate is treated with lithium hydroxide monohydrate to yield 4-acetyl-5-thiazolecarboxylic acid.

**BIOLOGICAL ACTIVITY**

Example I3

Pre-emergence herbicidal activity of selected compounds of the present invention was determined as follows: Seeds of selected weeds are planted and the soil was drenched with a solution of water (I7 %), surfactant (O.I7 %) and the test compound at a rate equivalent ot IO lb)/acre. Scoring was made two weeks after treatment. The grasses (GR) Setaria viridis, Echinochloa crus-galli, Sorghum bicolorand Avena fatua and the broadleafs (BL) Ipomoea purpurea, Brassica juncea, Solanum nigrum and Abutilon theophrasti were treated. The test results indicate pre-emergent herbicidal activity of the compounds of the invention.

Example I4

Post-emergence herbicidal activity of selected compounds of the present invention was tested as follows: Seedlings of selected weeds were sprayed with a solution of water/acetone (I:I), surfactant (O.5%) and the test compound at a rate equivalent to IO lb/acre. Scoring was made two weeks after spraying. The grasses (GR) Setaria viridis, Echinochloa crusgalli, Sorghum bicolor and Avena fatua and the broadleafs (BL) Ipomoea purpurea, Brassica juncea, soybean and Abutilon theophrastic were treated. The test results indicate post-emergent herbicidal activity of the compounds of the invention.

**COMPOSITION EXAMPLES**

Example I5

A. Water dispersible powder

The sodium salt or the isopropylammonium salt of Compound I3 is dissolved to the desired percentage concentration in water containing O.5% surfactant (e.g., a I:I:I mixture of sorbitan monolaurate : polyoxyethylene[2O]sorbitan monolaurate : polyoxyethylene[2O]sorbitan trioleate).

B. Suspension concentrate - 26 %

Compound I3 (acid form) 26 %
propylene glycol 73 %
octyl phenoxypoly[ethylene-oxy]ethanol. 1 %

The above components are mixed and wet-milled to 5-IO micron particle size.

C. Wettable powder - 5O %

sodium salt of Compound I3 5O %
sodium lignosulfonate 4 %
sodium dialkylnaphthalene sulfonate 1 %
kaolin 45 %.

The above components are mixed and dry-milled. The resulting mixture is added to water for spraying.

**Claims**

I. Compounds of formula (A)

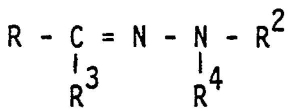 (A)

wherein R is an heteroaromatic group selected from the groups $G_1$ to $G_9$,

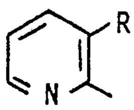
($G_1$)

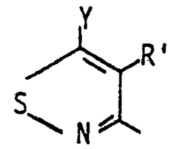
($G_2$)

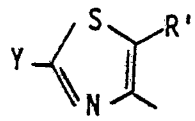
($G_3$)

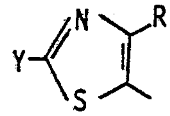
($G_4$)

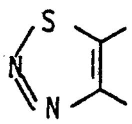
($G_5$)

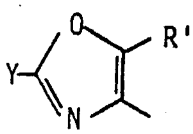
($G_6$)

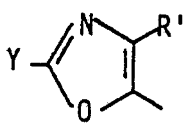
($G_7$)

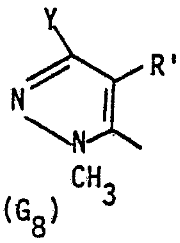
($G_8$)

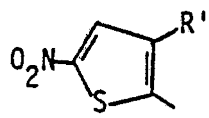
($G_9$)

in which R′ is the carboxy group in free form, salt form or ester form or is the group CO-$SR^6$ or CO-$NR^7R^8$, and Y is H, $C_{1-8}$alkyl, $C_{1-8}$haloalkyl, $C_{1-8}$alkoxy, $C_{1-8}$haloalkoxy, $C_{2-8}$alkenyloxy, $C_{2-8}$haloalkenyloxy, $C_{2-8}$alkynyloxy, phenyl, phenoxy, $C_{2-5}$alkenyl, $C_{1-8}$alkylthio, OH, halogen, nitro or cyano,
$R^2$ is the group -C(=$NR^{10}$)-$SR^9$ or -C(=X)-$NHR^{10}$,
$R^3$ is H or $C_{1-8}$alkyl,
$R^4$ is H, $C_{1-8}$alkyl or 2-hydroxyethyl,
$R^7$ and $R^8$, independently, are H or $C_{1-8}$alkyl or
$R^7$ together with $R^8$ is $-(CH_2)_2-O-(CH_2)_2-$,
each of $R^6$ and $R^9$ is, independently, $C_{1-8}$alkyl, $C_{2-8}$alkenyl, phenyl or benzyl,
X is oxygen or sulfur,
$R^{10}$ is one of the groups

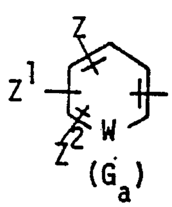
($G_a$)

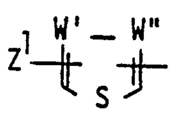
($G_b$)

in which W, W′ and W″, independently, are N or CH,
and Z, $Z^1$ and $Z^2$, independently, are one of the significances specified for - but independent of - Y with the provisos that either

a) where R is a nicotinyl acid group in free acid form, salt form or ester form, then $R^2$ is the group -C(=$NR^{10}$)-$SR^9$ or

b) where R is a nicotinyl acid group in free acid form, salt form or ester form and $R^2$ is the group -C(=X)-$NHR^{10}$, then $R^{10}$ is the group ($G_b$), or

c) where R is a nicotinyl acid group in free acid form, salt form or ester form, $R^2$ is the group -C(=X)-$NHR^{10}$ and $R^{10}$ is the group ($G_a$), then either

i) one of Z, $Z^1$ and $Z^2$ is selected from $C_{2-5}$alkenyl, $C_{1-8}$alkylthio and $C_{1-8}$haloalkoxy,

or

ii) $Z^1$ is $C_{1-8}$alkyl, $Z^2$ is halogen or $C_{1-8}$alkyl and Z is H, or

iii) $Z^1$ and $Z^2$ are both bromo, and Z is H, or

iv) $R^4$ is 2-hydroxyethyl.

2. The compound of Claim I, wherein R′ is the carboxy group in free form, in salt form or in the ester form

$COOR^5$,
Y is H, halogen, $C_{1-4}$alkyl or $C_{1-45}$alkoxy,
$R^3$ is $CH_3$ or $C_2H_5$,
$R^4$ is H, $CH_3$, $C_2H_5$ or 2-hydroxyethyl,
$R^5$ is $C_{1-4}$alkyl, phenyl, benzyl, allyl, $C_{1-4}$haloalkyl or $CH(R^{11})$-O-C-(O)-$R^{12}$,
$R^9$ is $C_{1-4}$alkyl, $C_{2-5}$alkenyl, phenyl or benzyl,
$R^{10}$ is either group $G_a$ in which
W is CH,
Z is H
$Z^1$ is H, $C_{1-8}$alkyl, $C_{2-5}$alkenyl, $C_{1-8}$alkylthio, $C_{1-8}$alkoxy, $C_{1-8}$haloalkoxy, halogen or $CF_3$,
and $Z^2$ is H, $C_{1-4}$alkyl, halogen or $C_{1-4}$alkoxy or is 2-thiazole or 5-($C_{1-4}$alkyl)-2-(I,3,4-thiadiazole),
$R^{11}$ is H or $CH_3$, and
$R^{12}$ is $C_{1-8}$alkyl.

3. The compound of Claim I or 2, wherein R is group $G_1$.

4. The compound of Claim I or 2, wherein R is group $G_9$.

5. The compound of Claim I or 2, wherein R is selected from the groups $G_2$ to $G_8$.

6. The compound of Claim 5, wherein R is group $G_2$.

7. The compound of Claim 3, wherein $R^2$ is the group -C(=$NR^{10}$)-$SR^9$.

8. The compound of Claim 4, wherein $R^2$ is the group -C(=$NR^{10}$)-$SR^9$.

9. The compound of Claim 5, wherein $R^2$ is the group -C(=$NR^{10}$)-$SR^9$.

IO. The compound of Claim 6, wherein $R^2$ is the group -C(=$NR^{10}$)-$SR^9$.

II. The compound of Claim 6, wherein
$R^3$ is $CH_3$ or $C_2H_5$,
$R^4$ is H, $CH_3$ or $C_2H_5$,
$Z^1$ is H, $C_{1-2}$alkyl, methoxy, halogen, $CF_3$ or $OCF_3$,
$Z^2$ is H, halogen, methyl or methoxy,
$R^2$ is C(X)-$NHR^{10}$,
$R^5$ is $CH(R^{11})$-O-C(O)-$R^{12}$,
$R^{11}$ is H, methyl, ethyl or sec. butyl,
and $R^{12}$ is H, methyl, ethyl, n-propyl, sec. butyl or tert. butyl.

I2. The compound of Claim 6, wherein $R^2$, $R^3$, $R^4$, $R^5$, $R^{11}$, $R^{12}$ and $Z^2$ are as defined in Claim II and $Z^1$ is $OCHF_2$ or $OCF_2$-$CHF_2$.

I3. The compound of Claim 6, wherein $R^2$, $R^3$, $R^4$ and $Z^2$ are as defined in Claim II, $Z^1$ is H, $C_{1-2}$alkyl, methoxy, halogen, $CF_3$, $OCF_3$, $OCHF_2$ or $OCHF_2$-$CHF_2$ and $R^5$ is $C_{1-4}$alkyl, $CH_2CF_3$ or phenyl.

I4. The compound of any one of Claims I to I3, wherein X is O.

I5. 3-Acetyl-4-isothiazolecarboxylic acid 4-(3-fluorophenyl)semicarbazone, in free acid form, salt form or ester form.

I6. The compound of Claim I5 in free acid form.

I7. The compound of Claim I5 in salt form.

I8. Process of preparing a compound of formula (A) stated in Claim I, which comprises

a) reacting a compound of formula I

R - C(=O)-$R^3$ I

wherein R and $R^3$ are as defined in Claim I
with a compound of formula II

$H_2N$ - $NR^2R^4$ II

wherein $R^2$ and $R^4$ are as defined in Claim I,
followed, where desired, by esterifying compounds of formula (A) wherein R′ is the carboxyl group to compounds (A) wherein R′ is a carboxyl-ester group, or

b) S-alkylating a compound of formula $A_1$

```
                    S
                    "
R - C = N - N - C - NHR10         (A1)
    |3      |4
    R       R
```

wherein R, $R^3$, $R^4$ and $R^{10}$ are as defined in Claim I,
with a halogenide of formula III

$R^9Hal$ III

wherein $R^9$ is as defined in Claim I,
Hal is halogen,
or a reactive functional derivative of said compound of formula III

to give a compound of formula ($A_2$)

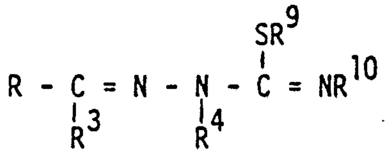

($A_2$)

wherein R, $R^3$, $R^4$, $R^9$ and $R^{10}$ are as defined in Claim 1,

and isolation thereby any compound of formula (A), wherein R′ is carboxy in free form or in salt form.

19. A herbicidal composition comprising a compound of formula (A) according to any one of Claims 1 to 17 and an agriculturally acceptable diluent.

20. A method of combatting weeds which comprises applying to the locus thereof a herbicidally effective amount of a compound of formula (A) according to any one of Claims 1 to 17.

21. The method of Claim 20 which comprises selectively combatting weeds in a crop locus.

22. The method of Claim 21 which comprises applying a compound of any one of Claims 6 and 11 to 17, to a corn (Maize) or soybean locus.

Claims for the following Contracting States: AT; ES

1. Herbicidal compositions comprising a compound of formula (A)

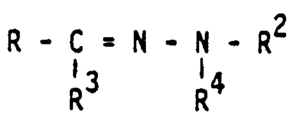

(A)

wherein R is an heteroaromatic group selected from the groups $G_1$ to $G_9$,

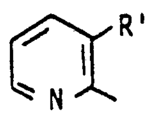
($G_1$)

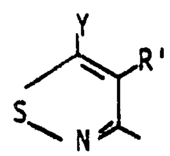
($G_2$)

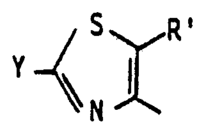
($G_3$)

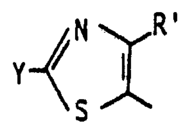
($G_4$)

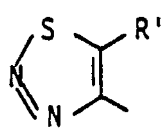
($G_5$)

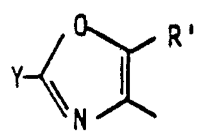
($G_6$)

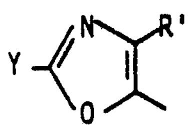
($G_7$)

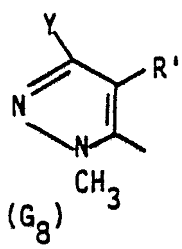
($G_8$)

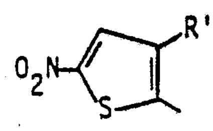
($G_9$)

in which R′ is the carboxy group in free form, salt form or ester form or is the group CO-$SR^6$ or CO-$NR^7R^8$, and Y is H, $C_{1-8}$alkyl, $C_{1-8}$haloalkyl, $C_{1-8}$alkoxy, $C_{1-8}$haloalkoxy, $C_{2-8}$alkenyloxy, $C_{2-8}$haloalkenyloxy, $C_{2-8}$alkynyloxy, phenyl, phenoxy, $C_{2-5}$alkenyl, $C_{1-8}$alkylthio, OH, halogen, nitro or cyano,

$R^2$ is the group $-C(=NR^{10})-SR^9$ or $-C(=X)-NHR^{10}$,

$R^3$ is H or $C_{1-8}$alkyl,

$R^4$ is H, $C_{1-8}$alkyl or 2-hydroxyethyl,

$R^7$ and $R^8$, independently, are H or $C_{1-8}$alkyl or

$R^7$ together with $R^8$ is $-(CH_2)_2-O-(CH_2)_2-$,

each of $R^6$ and $R^9$ is, independently, $C_{1-8}$alkyl, $C_{2-8}$alkenyl, phenyl or benzyl,

X is oxygen or sulfur,

$R^{10}$ is one of the groups

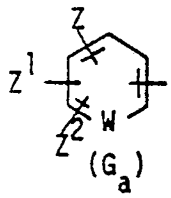

($G_a$)

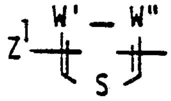

($G_b$)

in which W, W′ and W″, independently, are N or CH,
and Z, $Z^1$ and $Z^2$, independently, are one of the significances specified for - but independent of - Y
with the provisos that either

a) where R is a nicotinyl acid group in free acid form, salt form or ester form, then $R^2$ is the group $-C(=NR^{10})-SR^9$ or

b) where R is a nicotinyl acid group in free acid form, salt form or ester form, and $R^2$ is the group $-C(=X)-NHR^{10}$, then $R^{10}$ is the group ($G_b$), or

c) where R is a nicotinyl acid group in free acid form, salt form or ester form, $R^2$ is the group $-C(=X)-NHR^{10}$ and $R^{10}$ is the group ($G_a$), then either

i) one of Z, $Z^1$ and $Z^2$ is selected from $C_{2-5}$alkenyl, $C_{1-8}$alkylthio and $C_{1-8}$haloalkoxy,

or

ii) $Z^1$ is $C_{1-8}$alkyl, $Z^2$ is halogen or $C_{1-8}$alkyl and Z is H, or

iii) $Z^1$ and $Z^2$ are both bromo, and Z is H, or

iv) $R^4$ is 2-hydroxyethyl,

and an agriculturally acceptable diluent.

2. The composition of Claim I wherein R′ is the carboxy group in free form, in salt form or in the ester form $COOR^5$,
Y is H, halogen, $C_{1-4}$alkyl or $C_{1-45}$alkoxy,
$R^3$ is $CH_3$ or $C_2H_5$,
$R^4$ is H, $CH_3$, $C_2H_5$ or 2-hydroxyethyl,
$R^5$ is $C_{1-4}$alkyl, phenyl, benzyl, allyl, $C_{1-4}$haloalkyl or $CH(R^{11})-O-C(O)-R^{12}$,
$R^9$ is $C_{1-4}$alkyl, $C_{2-5}$alkenyl, phenyl or benzyl,
$R^{10}$ is either group $G_a$ in which
W is CH,
Z is H
$Z^1$ is H, $C_{1-8}$alkyl, $C_{2-5}$alkenyl, $C_{1-8}$alkylthio, $C_{1-8}$alkoxy, $C_{1-8}$haloalkoxy, halogen or $CF_3$,
and $Z^2$ is H, $C_{1-4}$alkyl, halogen or $C_{1-4}$alkoxy or is 2-thiazole or 5-($C_{1-4}$alkyl)-2-(l,3,4-thiadiazole),
$R^{11}$ is H or $CH_3$, and
$R^{12}$ is $C_{1-8}$alkyl.

3. The composition of Claim I or 2, wherein R is group $G_1$.

4. The composition of Claim I or 2, wherein R is group $G_9$.

5. The composition of Claim I or 2, wherein R is selected from the groups $G_2$ to $G_8$.

6. The composition of Claim 5, wherein R is group $G_2$.

7. The composition of Claim 3, wherein $R^2$ is the group $-C(=NR^{10})-SR^9$.

8. The composition of Claim 4, wherein $R^2$ is the group $-C(=NR^{10})-SR^9$.

9. The composition of Claim 5, wherein $R^2$ is the group $-C(=NR^{10})-SR^9$.

IO. The composition of Claim 6, wherein $R^2$ is the group $-C(=NR^{10})-SR^9$.

II. The composition of Claim 6 wherein
$R^3$ is $CH_3$ or $C_2H_5$,
$R^4$ is H, $CH_3$ or $C_2H_5$,
$Z^1$ is H, $C_{1-2}$alkyl, methoxy, halogen, $CF_3$ or $OCF_3$,
$Z^2$ is H, halogen, methyl or methoxy,
$R^2$ is $C(X)-NHR^{10}$,
$R^5$ is $CH(R^{11})-O-C(O)-R^{12}$,
$R^{11}$ is H, methyl, ethyl or sec. butyl,
and $R^{12}$ is H, methyl, ethyl, n-propyl, sec. butyl or tert. butyl,

I2. The composition of Claim 6, wherein $R^2$, $R^3$, $R^4$, $R^5$, $R^{11}$, $R^{12}$ and $Z^2$ are as defined in Claim II and $Z^1$ is $OCHF_2$ or $OCF_2-CHF_2$.

I3. The composition of Claim 6, wherein $R^2$, $R^3$, $R^4$ and $Z^2$ are as defined in Claim II, $Z^1$ is H, $C_{1-2}$alkyl, methoxy, halogen, $CF_3$, $OCF_3$, $OCHF_2$ or $OCHF_2-CHF_2$ and $R^5$ is $C_{1-4}$alkyl, $CH_2CF_3$ or phenyl.

I4. The composition of any one of Claims I to I3, wherein X is O.

I5. Herbicidal composition comprising
3-acetyl-4-isothiazolecarboxylic acid 4-(3-fluorophenyl)semicarbazone, in free acid form, salt form or ester form.

I6. The composition of Claim I5 comprising the compound defined in Claim I5 in free acid form.

17. The composition of Claim 15 comprising the compound defined in Claim 15 in salt form.

18. Process of preparing a compound of formula (A) stated in Claim 1, which comprises

a) reacting a compound of formula I

R - C(=O)-$R^3$ I

wherein R and $R^3$ are as defined in Claim I

with a compound of formula II

$H_2N$ - $NR^2R^4$ II

wherein $R^2$ and $R^4$ are as defined in Claim I,

followed, where desired, by esterifying compounds of formula (A) wherein R′ is the carboxyl group to compounds (A) wherein R′ is the carboxyl-ester group, or

b) S-alkylating a compound of formula $A_1$

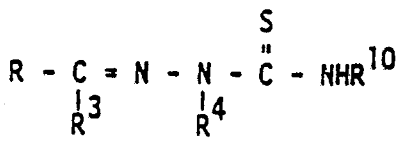

($A_1$)

wherein R, $R^3$, $R^4$ and $R^{10}$ are as defined in Claim I,

with a halogenide of formula III

$R^9$Hal III

wherein $R^9$ is as defined in Claim I,

Hal is halogen,

or a reactive functional derivative of said compound of formula III to give a compound of formula ($A_2$)

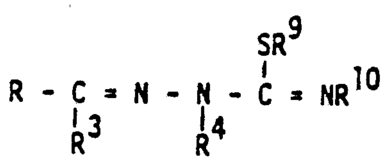

($A_2$).

wherein R, $R^3$, $R^4$, $R^9$ and $R^{10}$ are as defined in Claim I,

and isolation thereby any compound of formula (A), wherein R′ is carboxy in free form or in salt form.

19. A method of combatting weds which comprises applying to the locus thereof a herbicidally effective amount of a compound of formula (A) as defined in any one of Claims I to 17.

20. The method of Claim 19 which comprises selectively combatting weeds in a crop locus.

21. The method of Claims 20 which comprises applying a compound as defined in any one of Claims 6 and 11 to 17 to a corn (maize) or soybean locus.

European Patent Office

# EUROPEAN SEARCH REPORT

Application number

EP 87810444.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 4 317 776 (KLAYMAN et al.)<br>* Claim 1; column 8, lines 30-33 *<br>-- | 1,18 | C 07 D 275/02<br>C 07 D 277/30<br>C 07 D 277/34<br>C 07 D 285/06<br>C 07 D 231/16<br>C 07 D 213/80<br>C 07 D 213/82<br>C 07 D 213/83<br>C 07 D 263/32<br>C 07 D 263/38<br>C 07 D 263/48<br>C 07 D 401/12<br>C 07 D 403/12<br>C 07 D 409/12<br>C 07 D 413/12<br>C 07 D 417/12<br>C 07 D 333/44<br>A 01 N 43/40<br>A 01 N 43/80<br>A 01 N 43/78 |
| A | CHEMICAL ABSTRACTS, vol. 99, no. 7, August 15, 1983, Columbus, Ohio, USA<br>DEMILO, A.B.; REDFERN, R.E.; BORKOVEC, A.B.; "2-Acetylpyridine thiosemicarbazones as inhibitors of ecdysis in Oncopeltus fasciatus: structure-activity relationship study"<br>page 155, column 1, abstract-no. 48 910t<br>& J. Agric. Food Chem. 1983, 31(4), 713-18<br>-- | 1,18 | TECHNICAL FIELDS SEARCHED (Int. Cl.4) |
| A | CHEMICAL ABSTRACTS, vol. 98, no. 3, January 17, 1983, Columbus, Ohio, USA<br>KLAYMAN, D.L.; SCOVILL, J.P.; BARTOSEVICH, J.F.; BRUCE, J. "2-Acetylpyridine thiosemicarbazones. 5. 1-[1-(2-Pyridyl)ethyl] -3-thio-semicarbazides as potential anti-malarial agents"<br>page 499, column 2, abstract-no. 16 566y<br>& J. Med. Chem. 1983, 26(1), 35-9<br>-- | 1,18 | C 07 D 275/00<br>C 07 D 277/00<br>C 07 D 285/00<br>C 07 D 231/00<br>C 07 D 213/00<br>C 07 D 263/00<br>C 07 D 401/00<br>C 07 D 403/00<br>C 07 D 409/00<br>C 07 D 413/00<br>C 07 D 417/00<br>C 07 D 333/00 |
| A | GB - A - 1 306 933 (LILLY INDUSTRIES)<br>* Claims *<br>-- | 1 | |

The present search report has been drawn up for all claims

| Place of search | Date of completion of the search | Examiner |
|---|---|---|
| VIENNA | 12-11-1987 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number



EP 87810444.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE - B - 1 063 601 (LEPETIT)<br>* Totality *<br>---- | 1 | A 01 N 43/82<br>A 01 N 43/76<br>A 01 N 43/56<br>A 01 N 43/10 |
| | | | TECHNICAL FIELDS SEARCHED (Int. Cl.4) |

The present search report has been drawn up for all claims

| Place of search | Date of completion of the search | Examiner |
|---|---|---|
| VIENNA | 12-11-1987 | BRUS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document